# EUROPEAN PATENT SPECIFICATION

(11) **EP 1 851 320 B1**
(45) Date of publication and mention of the grant of the patent: **28.12.2011**
(21) Application number: 06703897.6
(22) Date of filing: 24.01.2006
(51) Int. Cl.: C12N 15/863, A61K 39/00, A61K 39/39

(54) **VACCINES BASED ON THE USE OF MVA**
AUF DER VERWENDUNG VON MVA BERUHENDE IMPFSTOFFE
VACCINS À BASE DE L'UTILISATION DE MVA

(30) Priority: 24.01.2005 EP 05001362
(43) Date of publication of application: 07.11.2007
(73) Proprietor: Helmholtz Zentrum München Deutsches Forschungszentrum für Gesundheit und Umwelt (GmbH), 85764 Neuherberg (DE)
(72) Inventor: DREXLER, Ingo, 80797 München (DE); SUTTER, Gerd, 80803 München (DE); GASTEIGER, Georg, 81669 München (DE); ERFLE, Volker, 81675 München (DE)
(74) Representative: Sandmann, Wolfgang
(86) International application number: PCT/EP2006/000608
(87) International publication number: WO 2006/077161

(56) References cited:
- WO-A-2004/093905
- WANG ZHONGDE ET AL: "Attenuated poxviruses generate clinically relevant frequencies of CMV-specific T cells" BLOOD, vol. 104, no. 3, 1 August 2004 (2004-08-01), pages 847-856, XP002333163 ISSN: 0006-4971 cited in the application
- DATABASE BIOSIS [Online] BIOSCIENCES INFORMATION SERVICE, PHILADELPHIA, PA, US; 16 November 2003 (2003-11-16), DIAMOND DON J ET AL: "Attenuated poxvirus expressing multiple CMV antigens is a highly efficient means to elicit human CD8 T lymphocytes suitable for adoptive immunotherapy or donor immunization." XP002333164 Database accession no. PREV200400181006 & BLOOD, vol. 102, no. 11, 16 November 2003 (2003-11-16), pages 40a-41a, 45TH ANNUAL MEETING OF THE AMERICAN SOCIETY OF HEMATOLOGY; SAN DIEGO, CA, USA; DECEMBER 06-09, 2003 ISSN: 0006-4971
- HANKE T ET AL: "Enhancement of MHC class I-restricted peptide-specific T cell induction by a DNA prime/MVA boost vaccination regime" VACCINE, BUTTERWORTH SCIENTIFIC. GUILDFORD, GB, vol. 16, no. 5, March 1998 (1998-03), pages 439-445, XP004106957 ISSN: 0264-410X
- EUR J IMMUNOL, vol. 37, 2007, pages 2063-2074,
- J. IMMUNOLOGY, vol. 174, 2005, pages 4373-4380,
- INTERNATIONAL J MEDICAL MICROBIOLOGY, vol. 298, 2008, pages 27-32,

### SEQUENCE LISTING

<110> GSF-Forschungszentrum für Umwelt und Gesundheit GmbH
<120> MVA vaccine
<130> P20168
<140> PCT
   <141> 2006-01-24
<150> EP 05001362.2
   <151> 2005-01-24
<160> 6
<170> PatentIn version 3.3
<210> 1
   <211> 40
   <212> DNA
   <213> Artificial
<220>
   <223> Primer
<400> 1
   gggcggatcc gaccatgcag atcttcgtga agaccctgac 40
<210> 2
   <211> 42
   <212> DNA
   <213> Artificial
<220>
   <223> Primer
<400> 2
   caaaacagcc aggagcatcg cacctctcag gcgaaggacc ag 42
<210> 3
   <211> 42
   <212> DNA
   <213> Artificial
<220>
   <223> Primer
<400> 3
   cgcctgagag gtgcgatgct cctggctgtt ttgtactgcc tg 42
<210> 4
   <211> 41
   <212> DNA
   <213> Artificial
<220>
   <223> Primer
<400> 4
   gggcgtttaa acttataaat ggctctgata caagctgtgg t 41
<210> 5
   <211> 40
   <212> DNA
   <213> Artificial
<220>
   <223> Primer
<400> 5
   gggcggatcc gaccatgcag atcttcgtga agaccctgac 40
<210> 6
   <211> 41
   <212> DNA
   <213> Artificial
<220>
   <223> Primer
<400> 6
   gggcgtttaa acttataaat ggctctgata caagctgtgg t 41

## Description

The present invention is in the field of vaccines based on recombinant modified vaccinia virus Ankara (MVA).

Vaccinia virus (VV) belongs to the genus Orthopoxvirus of the family of poxviruses. Certain strains of vaccinia virus have been used for many years as live vaccine to immunize against smallpox, for example the Elstree strain of the Lister Institute in the UK. Because of the complications which may derive from the vaccination (Schär, Zeitschr. für Präventivmedizin 18, 41-44 [1973]), and since the declaration in 1980 by the WHO that smallpox had been eradicated nowadays only people at high risk are vaccinated against smallpox.

Vaccinia viruses have also been used as vectors for production and delivery of foreign antigens (Smith et al., Biotechnology and Genetic Engineering Reviews 2, 383-407 [1984]). This entails DNA sequences (genes) which code for foreign antigens being introduced, with the aid of DNA recombination techniques, into the genome of the vaccinia viruses. If the gene is integrated at a site in the viral DNA which is non-essential for the life cycle of the virus, it is possible for the newly produced recombinant vaccinia virus to be infectious, that is to say able to infect foreign cells and thus to express the integrated DNA sequence (EP Patent Applications No. 83,286 and No. 110,385). The recombinant vaccinia viruses prepared in this way can be used, on the one hand, as live vaccines for the prophylaxis of infections, on the other hand, for the preparation of heterologous proteins in eukaryotic cells.

Vaccinia virus is amongst the most extensively evaluated live vectors and has particular features in support of its use as recombinant vaccine: It is highly stable, cheap to manufacture, easy to administer, and it can accommodate large amounts of foreign DNA. It has the advantage of inducing both antibody and cytotoxic responses, and allows presentation of antigens to the immune system in a more natural way, and it was successfully used as vector vaccine protecting against infectious diseases in a broad variety of animal models. Additionally, vaccinia vectors are extremely valuable research tools to analyze structure-function relationships of recombinant proteins, determine targets of humoral and cell-mediated immune responses, and investigate the type of immune defense needed to protect against a specific disease.

However, vaccinia virus is infectious for humans and its use as expression vector in the laboratory has been affected by safety concerns and regulations. Furthermore, possible future applications of recombinant vaccinia virus e.g. to generate recombinant proteins or recombinant viral particles for novel therapeutic or prophylactic approaches in humans, are hindered by the productive replication of the recombinant vaccinia vector. Most of the recombinant vaccinia viruses described in the literature are based on the Western Reserve (WR) strain of vaccinia virus. On the other hand, it is known that this strain is highly neurovirulent and is thus poorly suited for use in humans and animals (Morita et al., Vaccine 5, 65-70 [1987]).

Concerns with the safety of standard strains of VV have been addressed by the development of vaccinia vectors from highly attenuated virus strains which are characterized by their restricted replicative capacity *in vitro* and their avirulence *in vivo.* Strains of viruses specially cultured to avoid undesired side effects have been known for a long time. Thus, it has been possible, by long-term serial passages of the Ankara strain of vaccinia virus (CVA) on chicken embryo fibroblasts, to culture MVA (for review see Mayr, A., Hochstein-Mintzel, V. and Stickl, H. (1975) Infection 3, 6-14; Swiss Patent No. 568 392). The MVA virus was deposited in compliance with the requirements of the Budapest Treaty at CNCM (Institut Pasteur, Collectione Nationale de Cultures de Microorganisms, 25, rue de Docteur Roux, 75724 Paris Cedex 15) on Dec. 15, 1987 under Depositary No. I-721.

The MVA virus has been analysed to determine alterations in the genome relative to the wild type CVA strain. Six major deletions ( deletion I, II, III, IV, V, and VI ) have been identified (Meyer, H., Sutter, G. and Mayr A. (1991) J. Gen. Virol. 72, 1031-1038). This modified vaccinia virus Ankara has only low virulence, that is to say it is followed by no side effects when used for vaccination. Hence it is particularly suitable for the initial vaccination of immunocompromised subjects. The excellent properties of the MVA strain have been demonstrated in a number of clinical trials (Mayr et al., Zbl. Bakt. Hyg. I, Abt. Org. B 167, 375-390 [1987], Stickl et al., Dtsch. med. Wschr. 99,2386-2392 [1974]).

Modified vaccinia virus Ankara (MVA) is a valuable tool as safe viral vector for expression of recombinant genes and can be used for such different purposes as the *in vitro* study of protein functions or the *in vivo* induction of antigen-specific cellular or humoral immune responses. A major advantage of MVA is to allow for high level gene expression despite being replication defective in human and most mammalian cells. MVA as a vaccine has an excellent safety track-record, can be handled under biosafety level 1 conditions and has proven to be immunogenic and protective when delivering heterologous antigens in animals (1-8), and first human candidate vaccines have proceeded into clinical trials.

Although unable to multiply in most mammalian cell lines, MVA retains unimpaired expression of viral and heterologous genes (Sutter and Moss, 1992). Absence of pathogenicity for humans, inherent avirulence even in immunocompromised hosts, high-level expression of foreign antigens and adjuvant effect for immune responses make recombinant MVA (rMVA) an ideal vector for both prophylactic and therapeutic vaccination, as demonstrated by the wide use in prime-boost immunization strategies (Meseda et al., 2002; Amara et al., 2002) and in ongoing clinical trials (McConkey et al., 2003; Cosma et al., 2003; Hanke et al., 2002).

Indeed, recombinant MVA (rMVA)-based vaccines elicit both humoral and cell-mediated adaptive immune responses (Ramirez et al., 2000) and have proven to be protective in animal models of several infectious diseases (Hanke et al., 1999; Barouch et al., 2001; Weidinger et al., 2001; Schneider et al., 1998; Sutter et al., 1994b; Hirsch et al., 1996; Wyatt et al., 1996) and even in some tumor models (Carroll et al., 1997; Rosales et al., 2000; Drexler et al., 1999).

Standard procedures to generate rVV have been described in detail (Earl et al., 1998) and rely on *in vivo* homologous recombination between acceptor VV DNA and the transfected transfer plasmid, in which the foreign gene is flanked by VV sequences. Additional approaches for the generation of recombinant MVA are, for example, disclosed in WO 04074493, WO 03023040 and WO 9702355.

WANG et al., Blood, August 2004, Vol. 104, No. 3 discloses immunotherapeutic approaches to limit cytomegalovirus (CMV) morbidity and mortality after hematopoietic stem cell transplants. One approach comprises the attempt to insert ubiquitin-modified CMV-antigens into the virulent Western Reserve strain of vaccinia virus (VV) and the highly attentuated strain, modified vaccinia virus Ankara (MVA). Ubiquitin-modified CMV-antigens were phosphoproteins 65 (pp65), phosphoprotein 150 (pp150) and immediate early protein 1 (IE1) immunodominant antigens.

However, WANG et al. showed that antigen ubiquitination had no or only minor impact on primary immunity to CMV-antigens carried in rMVA.

Although modified vaccinia virus Ankara is regarded as being a valuable and safe viral vector for expression of recombinant genes, it is widely accepted that upper limits for the administration of MVA to mammals, in particular, human beings are existing. For example, the administration of recombinant MVA to humans is currently limited to 5x10⁸ IU (infectious units) per administration. This, however, may be disadvantageous in that the immune response generated by rMVA in this case might be insufficient in order to achieve the desired therapeutic effect. Thus, reducing the number of infectious units required to achieve a certain immune response in a mammal would be highly desirable.

Therefore, an object underlying the present invention is to provide a vaccination system based on MVA, which achieves sufficiently high immune responses in mammals, in particular, human beings, with a comparably low or reduced number of infectious units. It is a further problem underlying the present invention to provide an improved boosting agent for MVA based vaccination protocols, leading to an enhanced secondary immune response against an antigen of choice. Priming and boasting with MVA encoding an antigen per se are known in the art. See vaccine vol. 16, 1998, 439-445.

These problems are solved by the subject-matter of the independent claims. Preferred embodiments are set forth in the dependent claims.

In summary, the present invention brings about the following advantages and results:

It unexpectedly turned out that a combination of different kinds of recombinant MVA particles in a vaccination protocol, i.e. prime-boost vaccination protocols, showed enhanced cellular immune responses compared to the prior art approaches. In other words, it turned out that a combination of foreign protein for priming and recombinant MVA producing ubiquitinated foreign protein led to enhanced immune responses and needed less infectious units of rMVA to be administered to the mammals in order to achieve a sufficient immune response. These results were surprising since other scientists, for example, WANG et al. in the above mentioned publication in Blood, Volume 104, No. 3, indicated that antigen ubiquitination did not have an impact on the immunogenicity of rMVA.

In the present invention, it was confirmed that primary immune responses of rMVA carrying ubiquitinated foreign proteins were comparably low, however, large immune responses could be shown when said rMVA were used as a boosting agent. The inventors found out that by using the vaccination system of the present invention, the overall amount of infectious units of rMVA used in the vaccination can be dramatically reduced.

At first, the inventors constructed an ubiquitin/tyrosinase fusion gene (Ub-Tyr) by means of hybridization-PCR (Figure 1b). Then, via homologous recombination and subsequent host cell selection, recombinant MVA viruses could be successfully generated, in which the fusion gene was stably integrated in the viral genome and Ub-Tyr was produced as ubiquitinated fusion protein (Figure 1a). *In vitro,* the ubiquitination led to cytoplasmatic instability of the target proteins by rapid and nearly complete proteasome-dependent degradation, leading to a significant reduced half life of the ubiquitinated fusion protein (Figure 2). As mentioned above, by examining the immunogenicity *in vivo*, it surprisingly turned out that the MVA vaccine containing the ubiquitinated antigen showed a weak primary response compared to the MVA not containing ubiquitinated antigen, however, showed significantly enhanced secondary immune responses (see Figures 4 and 5).

In this application a new approach to optimize the generation of CTL using rMVA vectors by producing antigens designed for rapid proteasomal degradation to enhance peptide processing for MHC class I-specific presentation is claimed. MVA vaccines expressing an ubiquitin/foreign protein fusion protein were constructed and characterized *in vitro* by Western blot, Radio-Immuno-Precipitation and Chromium Release Assay.

*In vivo*, vaccination studies were carried out in HLA-A*0201-transgenic mice and CTL responses analyzed by intracellular cytokine synthesis and tetramer binding assays. *In vitro,* MVA-produced ubiquitinated tyrosinase (Ub-Tyr) was subject to rapid degradation which was specifically inhibited in the presence of proteasome inhibitors. Furthermore, ubiquitination resulted in significantly enhanced foreign protein-specific CTL recognition of infected target cells indicating increased peptide processing and availability of higher MHC class I-peptide densities on the cell surface even at earlier timepoints of the viral infection. In prime-boost vaccination studies, MVA-Ub-foreign protein was able to most efficiently enhance Tyr-specific CTL recall responses, importantly already at low doses of the vaccine.

The data presented herein show that MVA vaccines delivering distinct formulations of antigen could be selectively used for priming or boosting which is highly important for the development of optimized MVA-based vaccination protocols with improved immunogenicity.

According to a first aspect, the present invention is a medicament including a kit of parts comprising the following components:
a) a first component comprising one or more foreign proteins or a nncleic acid encoding same or functional parts thereof, which functional parts contain one or more substitutions, insertions and/or deletions when compared to the wild type protein whilst having the same flmction as the wild type protein; and
b) a second component comprising a recombinant Modified Vaccinia Virus Ankara (MVA), which carries a nucleic acid sequence coding for a fusion protein comprising:
   - Ubiquitin or a functional part thereof, which functional part contains one or more substitutions, insertions and/or deletions when compared to the wild type protein whilst having the same flrnction as the wild type protein; and
   - one or more foreign proteins, i.e. one or more proteins not naturally present as a part of MVA or functional parts thereof, which functional part contains one or more substitutions, insertions and/or deletions when compared to the wild type protein whilst having the same function as the wild type protein;
wherein the one or more foreign proteins are the same in a) and b), and wherein the first and second components further comprise a pharmaceutically acceptable carrier.

It is noted that the foreign protein employed in the two components is the same.

The term "foreign protein" as used herein also encompasses the alternative to include not only one, but also two or more distinct foreign proteins in one recombinant MVA particle. Thus, immunogenicity against several diseases may be achieved by only one vaccination. The use of two or more foreign proteins may assist also in avoiding or defeating escape mechanisms of certain viral and tumor diseases.

Furthermore, the term "foreign protein" is directed to a protein which is not naturally present as a part of MVA, i.e. a heterologous protein (and the nucleic acid encoding same).

Ubiquitin is a small, cytosolic protein, which is highly conserved. It plays a basic role in the organism in the regulation in the controlled degradation of proteins in the cells.

The polypeptide chain of ubiquitin consists of 76 amino acids. However, the term "ubiquitin" as used in the present invention is not restricted to this precise protein. This term also comprises and includes proteins of the protein superfamily called "ubiquitin-like proteins", i.e. proteins, which are showing a ubiquitin-like folding motif, as well as fragments and fusion proteins thereof. Thus, the invention also comprises an ubiquitin protein selected from proteins of the protein superfamily of the ubiquitin-like proteins, which proteins were modified by substitution, insertion, deletion, chemical modification all the like, however, which are retaining their specific folding motif or which result in introduction of the protein of interest into the cellular degradation machinery.

Examples of further proteins to be used in this respect are small Ub-like modifier (SUMO; see Yun-Cai Liu, Annu. Rev. Immunol. 2004. 22:81-127), PEST sequences (Duane A. Sewell et al., CANCER RESEARCH 64, 8821-8825, December 15, 2004). For further information, see also Chien-Fu Hung et al., CANCER RESEARCH 63, 2393-2398, May 15, 2003).

As explained above, the term "functional part" as used herein means such proteins or the nucleic acid encoding same, which contain one or more substitutions, insertions and or deletions when compared to the wild type protein/nucleic acid without altering its function. These lack preferably one, but also 2, 3, 4, or more nucleotides 5' or 3' or within the nucleic acid sequence, or these nucleotides are replaced by others. The "functional part" of a foreign protein may also be a truncated protein as long as it fulfills its physiological function, i.e. providing an immunogenicity which is effective in the treatment and/or protection of a specific disease. Thus, the foreign protein as used in the present invention could also be regarded as an "antigen" in the meaning which is common in the pertinent field of the art. An antigen herein is defined as a substance recognized by the immune system as foreign or toxic which elicits an immune response.

The foreign protein or antigen used in step a) is present in form of a nucleic acid, from which the foreign protein itself is expressed in the host, in form of recombinant bacteria expressing the foreign protein, of foreign protein in protein form and/or in form of foreign protein carried by a viral vector.

In a preferred embodiment, the viral vector is modified vaccinia virus Ankara (MVA).

According to a preferred embodiment, the fusion protein further comprises a linker between Ubiquitin and the foreign protein. The linker preferably comprises amino acids which either enhance the stability of the ubiquitin/protein fusion e.g. alanin at position 76 in the ubiquitin part of the fusion protein or amino acids which lead to preferred cleavage of the ubiquitin part e.g. glycine at position 76 in the ubiquitin part of the fusion protein thereby revealing amino acids contained within the fusion protein which lead to enhanced degradation of this part of the protein e.g. arginin at position 1 of the remaining part of the fusion protein and / or amino acids which contain amino acids wich serve as recognition signals to be targeted by further ubiquitin molecules within the cell thereby leading to enhanced degradation.

As used herein, the term "recombinant MVA" means those MVA, which have been genetically altered, e.g. by DNA recombination techniques and which are provided for the use, for example, as a vaccine or as an expression vector.

According to the present invention, the recombinant MVA vaccinia viruses can be prepared by several well-known techniques, for example the K1L-gene based selection protocol. As an example, a DNA-construct which contains a DNA-sequence which codes for the Vaccinia Virus (VV) *K1L* protein or a *K1L*-derived polypeptide and a DNA sequence encoding a foreign protein (or a fusion protein of Ubiquitin/foreign protein) both flanked by DNA sequences flanking a non-essential site, e. g. a naturally occuring deletion, e.g. deletion III, within the MVA genome, is introduced into cells, preferably eucaryotic cells. Preferably, avian, mammalian and human cells are used. Preferred eucaryotic cells are BHK-21 (ATCC CCL-10), BSC-1 (ATCC CCL-26), CV-1 (ECACC 87032605) or MA104 (ECACC 85102918) cells) productively infected with mutant MVA wherein the *K1L* gene sequences and its promoter sequences in the MVA genome or a functional part of said sequences have been inactivated, to allow homologous recombination. Further preferred host cells are chicken fibroblast cells, quail fibroblast cells, QT-9 cells, Vero cells, MRC-5 cells, B-cells or human primary cells (e.g. primary fibroblast cells, dendritic cells). For more detailed information see WO 04074493, which is incorporated herein in its entirety.

Once the DNA-construct has been introduced into the eukaryotic cell and the *K1L* coding DNA and foreign DNA has recombined with the viral DNA, it is possible to isolate the desired recombinant vaccinia virus MVA upon passage in cells that require *K1L* function to support virus growth, e.g. RK-13 cells. The cloning of the recombinant viruses is possible in a manner known as plaque purification (compare Nakano et al., Proc. Natl. Acad. Sci. USA 79, 1593-1596 [1982], Franke et al., Mol. Cell. Biol. 1918-1924 [1985], Chakrabarti et al., Mol. Cell. Biol. 3403-3409 [1985], Fathi et al., Virology 97-105 [1986]).

The DNA-construct to be inserted can be linear or circular. A circular DNA is preferably used. It is particularly preferable to use a plasmid.

The DNA-construct may contain sequences flanking the left and the right side of a non-essential site, e.g. the site of deletion III, within the MVA genome (Sutter, G. and Moss, B. (1992) Proc. Natl. Acad. Sci. USA 89, 10847-10851), the site of the engineered *K1L* deletion within the MVA genome or any non-essential site within the genome of mutant MVA according to this invention.

The foreign DNA sequence may be inserted between the sequences flanking the non-essential site, e.g. the naturally occurring deletion.

The foreign DNA sequence can be a gene coding for a therapeutic polypeptide, e.g secreted proteins, e.g. polypeptides of antibodies, chemokines, cytokines or interferons, or a polypeptide from a pathogenic agent which can be used preferably for vaccination purposes or for the production of therapeutic or scientific valuable polypeptides. Pathogenic agents are to be understood to be viruses, bacteria and parasites which may cause a disease, as well as tumor cells which multiply unrestrictedly in an organism and may thus lead to pathological growths. Examples of such pathogenic agents are described in Davis, B.D. et al., (Microbiology, 3rd ed., Harper International Edition). Preferred genes of pathogenic agents are those of influenza viruses, of measles and respiratory syncytial viruses, of dengue viruses, of human immunodeficiency viruses, for example HIV I and HIV II, of human hepatitis viruses, e.g. HCV and HBV, of herpes viruses, of papilloma viruses, of the malaria parasite Plasmodium falciparum, and of the tuberculosis-causing Mycobacteria.

Preferred genes encoding tumor associated antigens are those of melanoma-associated differentiation antigens, e.g. tyrosinase, tyrosinase-related proteins 1 and 2, of cancer testes antigens, e.g. MAGE-1,-2,-3, and BAGE, of non-mutated shared antigens overexpressed on tumors, e.g. Her-2/neu, MUC-1, and p53.

Further foreign sequences of use in the present invention are polypeptide sequences contained of artificial epitopes or epitope containing sequences e.g. minigenes, polytopes etc.

The DNA-construct can be introduced into the cells by transfection, for example by means of calcium phosphate precipitation (Graham et al., Virol. 52, 456-467 [1973]; Wigler et al., Cell 777-785 [1979]), by means of electroporation (Neumann et al., EMBO J. 1, 841-845 [1982]), by microinjection (Graessmann et al., Meth. Enzymology 101, 482-492 [1983]), by means of liposomes (Straubinger et al., Methods in Enzymology 101, 512-527 [1983]), by means of spheroplasts (Schaffner, Proc. Natl. Acad. Sci. USA 77, 2163-2167 [1980]) or by other methods known to those skilled in the art. Transfection by means of calcium phosphate precipitation is preferably used.

To prepare vaccines, the recombinant MVA of the present invention are converted into a physiologically acceptable form and are then combined in the kit of parts. This can be done based on the many years of experience in the preparation of vaccines used for vaccination against smallpox (Kaplan, Br. Med. Bull. 25, 131-135 [1969]). Typically, about 10⁶-10⁸ particles of the recombinant MVA are freeze-dried in 100ml of phosphate-buffered saline (PBS) in the presence of 2% peptone and 1% human albumin in an ampoule, preferably a glass ampoule. Further information regarding the dosage of MVA to be administered is indicated below. The lyophilisate can contain extenders (such as mannitol, dextran, sugar, glycine, lactose or polyvinylpyrrolidone) or other aids (such as antioxidants, stabilizers, etc.) suitable for parenteral administration. The glass ampoule is then sealed and can be stored, preferably at temperatures below -20°C, for several months.

For vaccination the lyophilisate can be dissolved in 0.1 to 0.2 ml of aqueous solution, preferably physiological saline, and administered parenterally, for example by intradermal inoculation. The vaccine according to the invention is preferably injected intracutaneously. Slight swelling and redness, sometimes also itching, may be found at the injection site (Stickl et al., supra). The mode of administration, the dose and the number of administrations can be optimized by those skilled in the art in a known manner. It is expedient where appropriate to administer the vaccine several times over a lengthy period in order to obtain a high level immune responses against the foreign antigen.

According to a preferred embodiment, in the kit of the present invention, the ratio of the amount of the first component to the second component is from 1:5 to 1:20, preferably 1:10, measured as infectious units (IU) of recombinant MVA particles. For example, the IU of recombinant MVA (not containing ubiquitin) used in the priming step is 10⁵-10⁶ for one mouse. This is a comparably low amount of particles compared to the approaches of the prior art. The dosage used for boosting the animal with recombinant MVA (containing ubiquitin) is about 10⁶-10⁷ for achieving the same T cell response as 10⁷-10⁸ IU of recombinant particles not containing ubiquitin (boosting step prior art). Thus, the number of IU used in the boosting step can be reduced by approximately 90% regarding the prior art techniques.

The upper limit for the administration of recombinant MVA to human beings is about 5 x 10⁸ IU, and an enhanced immune response may be achieved by only using an amount of recombinant MVA significantly lower than this upper limit.

According to a second aspect, the kit of parts as defined herein is intended for use in the anti-cancer therapy or in the prevention of infectious diseases.

According to a third aspect, the present invention is further directed to the use of a kit of parts as defined herein for the manufacture of a medicament for use in a method for enhancing T cell responses in a mammal, the method comprising the steps of:
a) Providing a kit as defined herein;
b) priming a mammal with an amount of the first component effective to provide a primary immune response;
c) boosting said mammal with an amount of the second component effective to provide a secondary immune response.

The kit may preferably be used for the manufacture of a medicament for treating cancer or for the prevention of infectious diseases.

According to a further preferred embodiment the boosting step is performed at week 2 - 12, preferably 4-8 after the priming step. As mentioned above, the kit of parts as disclosed herein is used in a vaccination method.

Preferably, the mammal treated is a human being.

In an alternative method, dendritic cells (DC) are isolated from patients or generated *ex vivo* and than infected with rMVA expressing ubiquitinated antigen according to the invention. These infected DC can be than adoptively transferred back into the patients as a vaccine in order to either directly prime naïve T cells or to expand existing T cells specific for the respective antigen. These infected DC can also be used to prime or expand T cells *in vitro* in order to adoptively transfer these *in vitro* generated T cells into the recipient.

According to a further aspect, the present invention is directed to
a recombinant MVA, which carries a nucleic acid sequence coding for a fusion protein comprising:
- Ubiquitin or a functional part thereof, which functional parts contain one or more substitutions, insertions and/or deletions when compared to the wild type protein whilst having the same function as the wild type protein; and
- one or more foreign proteins, i.e. one or more proteins not naturally present as a part of MVA or a functional part thereof which functional part contains one or more substitutions, insertions and/or deletions when compared to the wild type protein whilst having the same function as the wild type protein,
for use in either anti-cancer therapy or in the prevention of infectious diseases as a boosting agent in prime-boost vaccinations.

As mentioned above, it surprisingly turned that this recombinant MVA is showing enhanced secondary immune responses independent from the foreign protein involved.

The materials, methods, and examples are illustrative only.

The invention is now further illustrated by the accompanying drawings, in which the following is shown:

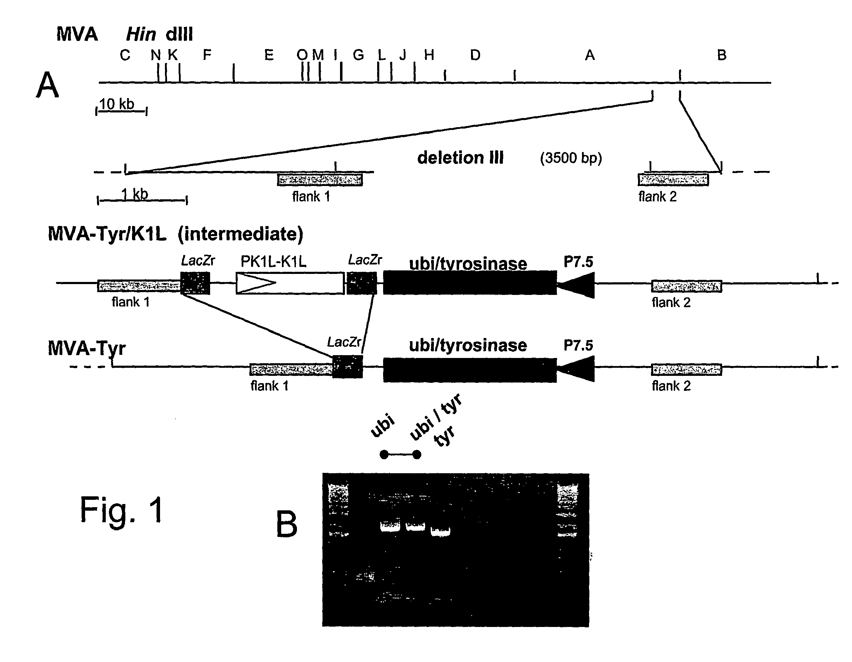

**FIG 1****.** Construction of recMVA expressing an ubiquitin/tyrosinase fusion gene under control of the vaccinia virus-specific promoter P7.5. (A) Schematic map of the insertion site into the viral genome (deletion III), and viral intermediate and final constructs obtained after homologous recombination. (B) Recombinant ubiquitin/tyrosinase fusion gene obtained after hybridization PCR (Agarose-Gel). As a control, single DNA fragments of ubiquitin or tyrosinase are also shown.

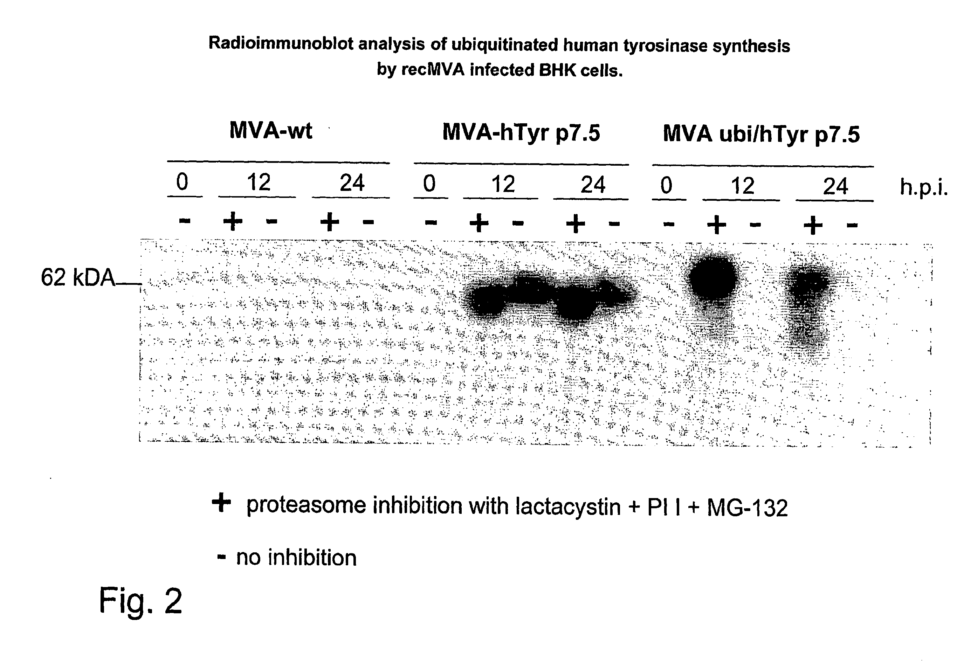

**FIG 2****.** Westernblot analysis of human *tyrosinase* gene expression in BHK cells infected with recMVA encoding authentic (MVA-hTyr P7.5) or ubiquinated tyrosinase (MVA-ubi/hTyr P7.5) or parental MVA (MVA-wt). Cells were harvested at the indicated hours post infection (h.p.i.). Cell lysates were separated by 8% SDS-PAGE. The blot from this gel was probed with anti-tyrosinase mAb T311 and peroxidase-labeled anti-mouse IgG secondary antibody, and visualized by enhanced chemoluminescence (ECL).

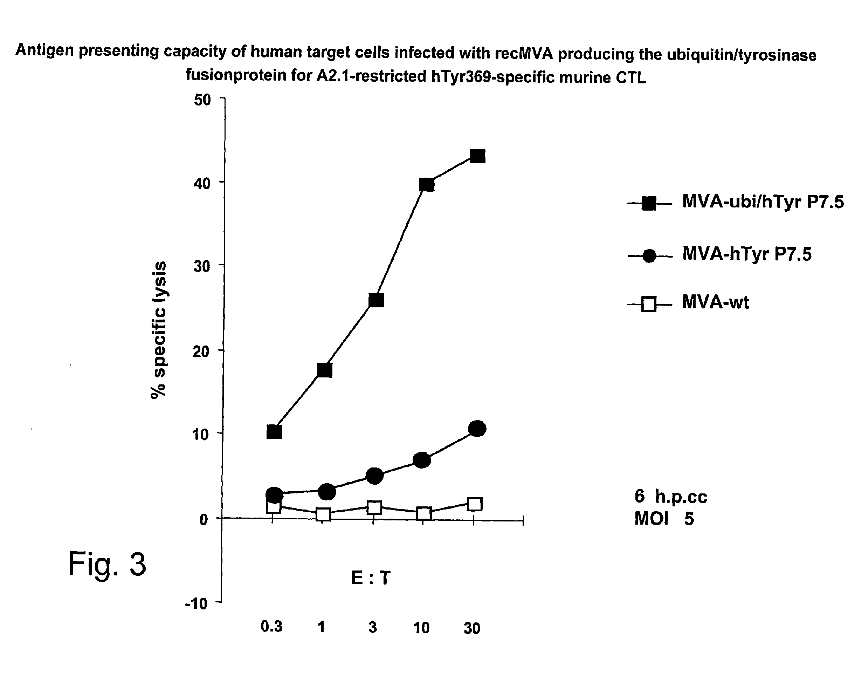

**FIG 3****.** Antigen presenting capacity of target cells infected with recMVA expressing human tyrosinase under control of the vaccinia virus-specific promoter P7.5 (MVA-hTyr P7.5 (•)) or in an ubiquitinated form (MVA-ubi/hTyr P7.5 (■)). Specific lysis by A*201-restricted murine CTL reactive against human tyrosinase peptide epitope 369-377 was determined in a 6-h [⁵¹Cr]-release assay.

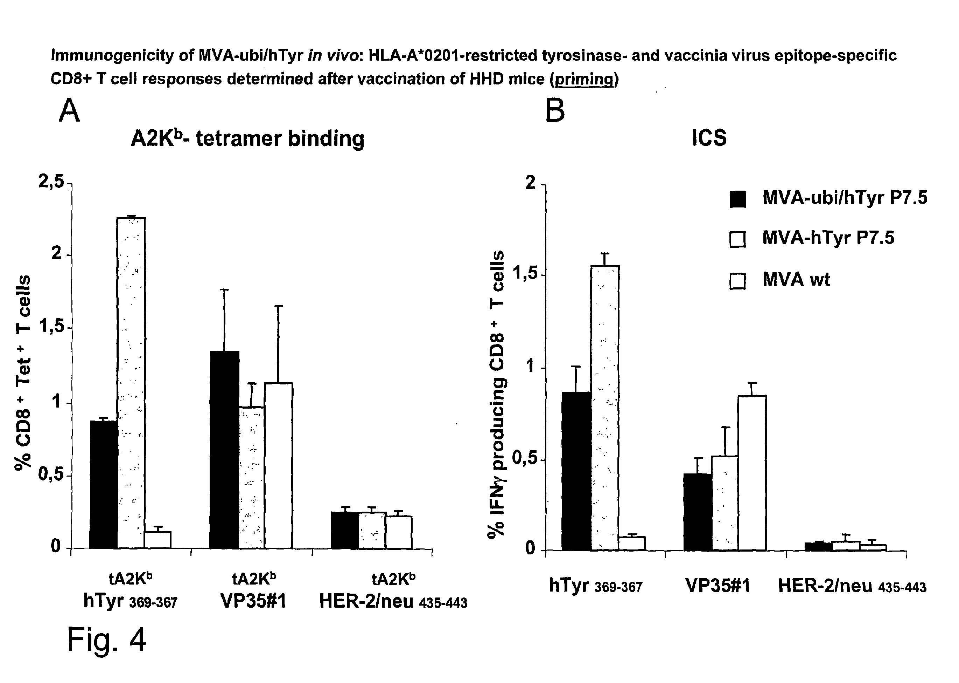

**FIG 4****.** Acute phase tyrosinase-specific primary CD8+ T cell responses induced by single immunization with recMVA viruses producing authentic or ubiquitinated tyrosinase. HHD-mice were primed i.p. with 10⁷ IU of MVA-hTyr P7.5 (grey bar), MVA-ubi/hTyr P7.5 (black bar) or MVA-wt (white bar). MVA- and tyrosinase-specific T-cell responses were analyzed on day 8. (A) Splenocytes were stained with chimeric A2Kb-tetramers specific for the human tyrosinase epitope 369-377, the VV epitope VP35#1 or the HER-2 epitope 435 as a control. (B) Splenocytes were peptide stimulated with either the human tyrosinase epitope 369-377, the VV epitope VP35#1 or the HER-2 epitope 435 as a control and than screened for intracellular Interferon γ production (ICS). All results are expressed as mean ± s.d. for four mice.

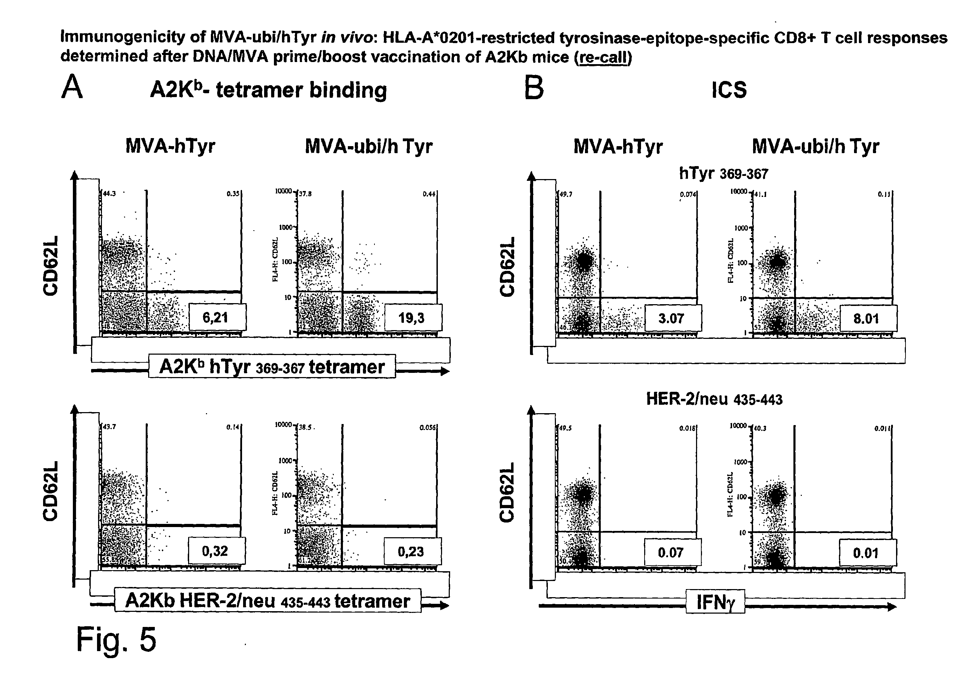

**FIG 5****.** Acute phase tyrosinase-specific secondary CD8+ T cell responses induced by heterologous DNA-MVA prime-boost immunization. A2Kb-mice were primed twice i.m. with DNA-vaccine encoding tyrosinase and boosted once i.p. with 10⁷ IU of recMVA producing authentic (MVA-hTyr) or ubiquitinated tyrosinase (MVA-ubi/hTyr). Splenocytes were analyzed on day 5 after the last vaccination for specificity for the human tyrosinase epitope 369-377 either by chimeric A2Kb-tetramerstaining (A) or ICS (B). Results are representative for at least three independent experiments.

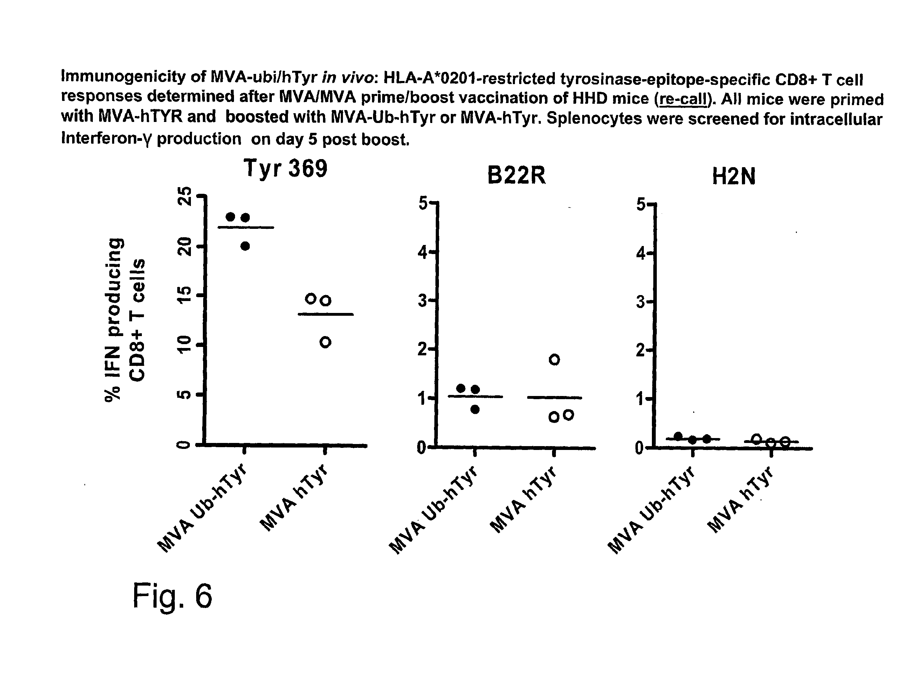

**FIG 6****.** Acute phase tyrosinase-specific secondary CD8+ T cell responses induced by heterologous MVA-MVA prime-boost immunization. HHD-mice were primed i.p. with 10⁷ IU of recMVA producing authentic (MVA-hTyr) tyrosinase and boosted i.p. with 10⁸ IU of recMVA producing authentic (MVA-hTyr) or ubiquitinated tyrosinase (MVA-ubi/hTyr). Splenocytes were harvested on day 5 after the last vaccination, peptide stimulated with either the human tyrosinase epitope 369-377, the VV epitope B22R or the HER-2 epitope 435 as a control and than screened for intracellular Interferon γ production (ICS)

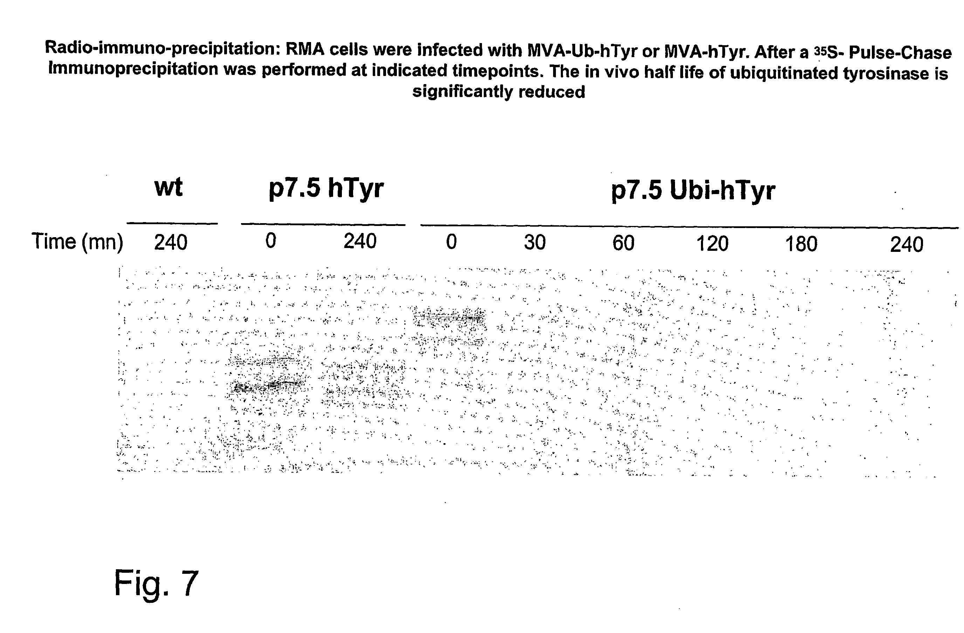

**FIG 7****.** Pulse-chase-experiment of RMA cells infected with recMVA encoding authentic (MVA-hTyr P7.5) or ubiquinated tyrosinase (MVA-ubi/hTyr P7.5) or parental MVA (MVA-wt). 5 hours post infection cells were starved for 20 min and then pulsed for 45 min with 50µCi of ³⁵S-labeled Methionine and Cystein, and then chased with RPMI medium. Immunoprecipitation was performed at indicated timepoints with anti-tyrosinase mAb C-19. Precipitates were separated by 8% SDS-PAGE and visualized on a phosphorimager. The *in vivo* half life of ubiquitinated tyrosinase is significantly reduced and can be estimated to be less than 30 min, whereas the *in vivo* half life of authentic tyrosinase has been estimated to be greater than 10 hrs (Jiménez et al., 1988).

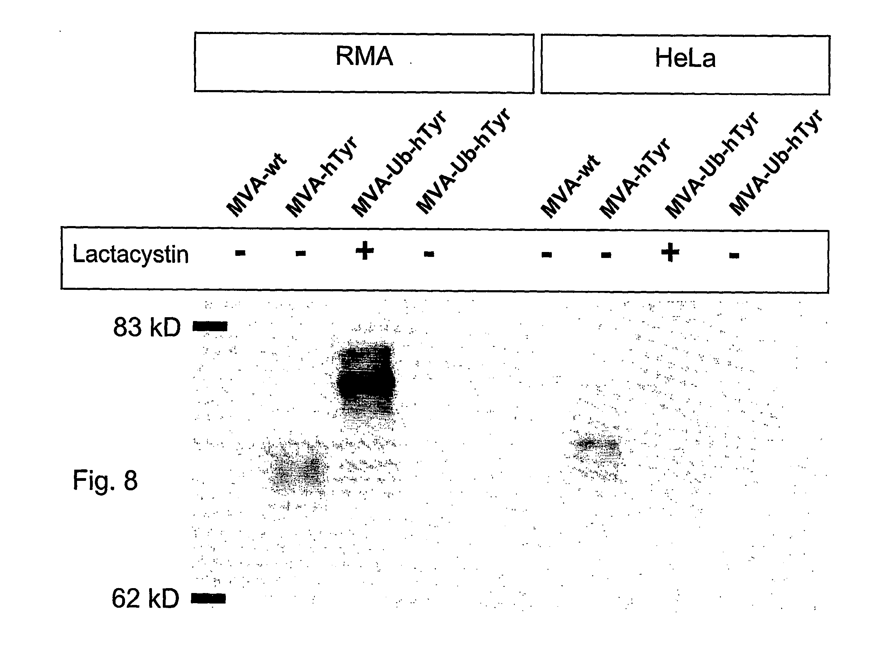

**Fig 8****.** Immunoprecipitation of ubiquitinated human tyrosinase expressed by recMVA in infected RMA and HeLa cells.

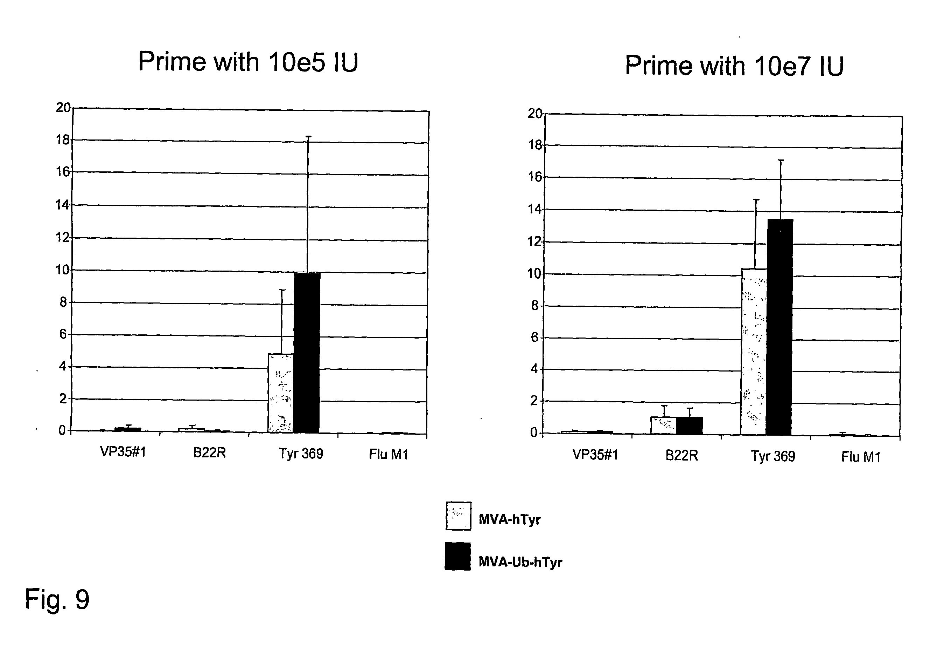

**Fig 9****.** Immunogenicity of MVA-ubi/hTyr *in vivo*: HLA-A*0201-restricted tyrosinase-epitope-specific CD8+ T cell responses determined after MVA/MVA prime/boost vaccination of HHD mice (re-call). All mice were primed with MVA-hTYR with either 10e5 IU (A) or 10e7 IU (B) and boosted with MVA-Ub-hTyr or MVA-hTyr at 10e7 IU. Splenocytes were screened for intracellular Interferon-γ production on day 5 post boost.

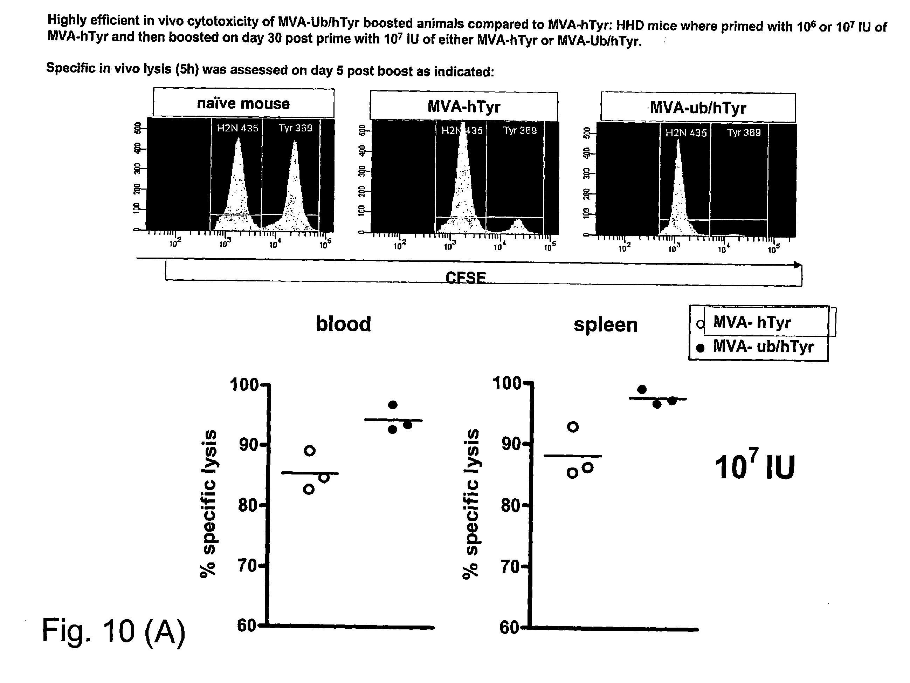

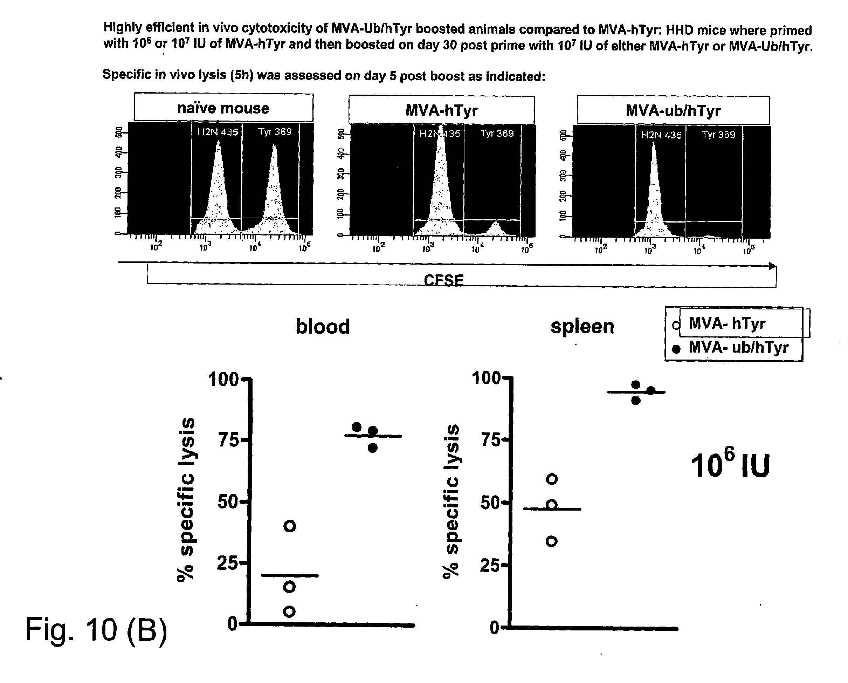

**Fig 10****.** Highly efficient in vivo cytotoxicity of MVA-Ub/hTyr compared to MVA-hTyr boosted animals after comparably low dose primary vaccinations. HHD mice where primed with 10⁷ (A) or 10⁶ (B) IU of MVA-hTyr and then boosted on day 30 post prime with 10⁷ IU of either MVA-hTyr or MVA-Ub/hTyr. Rapid in vivo killing of i.v. injected autologous spleenocytes labeled with the human tyrosinase epitope 369-377 or the HER-2 epitope 435-443 as a control was assessed on day 5 post boost. Specific 5h-in-vivo-lysis of target cells in the blood or the spleen is compared for different priming doses as indicated.

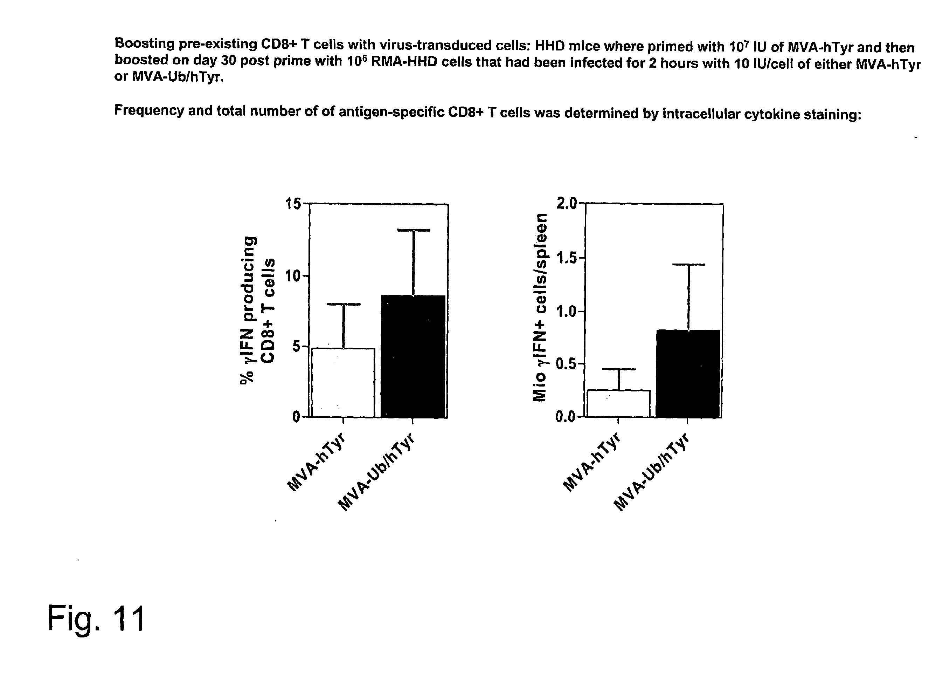

**Fig 11****.** Acute phase tyrosinase-specific secondary CD8+ T cell responses boosted by immunization with cells that were transduced with recMVA prior to immunization. HHD-mice were primed i.p. with 10⁷ IU of recNWA producing authentic (MVA-hTyr) tyrosinase and boosted i.p. with 10⁶ RMA-HHD cells that were infected wit 10 IU/cell of recMVA producing authentic (MVA-hTyr) or ubiquitinated tyrosinase (MVA-ubi/hTyr). Spleenocytes were harvested on day 5 after the last vaccination, peptide stimulated with the human tyrosinase epitope 369-377 or the HER-2 epitope 435-443 as a control and screened for intracellular Interferon γ production (ICS).

### Examples:

### Material and Methods

### Plasmid Construction

A ubiquitin/tyrosinase fusion gene was constructed to be cloned into the MVA transfer vector pIIIdHR-P7.5. Here we established a hybridisation-PCR-technique where the ubiquitin (Ub) gene could be fused to the tyrosinase (hTyr) cDNA without insertion of any additional non-Ub or non-hTyr DNA sequences. As ubiquitin expressed as a fusion to proteins is cleaved by cytosolic proteases at its G76 residue we aimed to mutate G76 to A76 which has been shown to prevent cytosolic cleavage when expressed by a plasmid vector (Rodriguez F *et al*, J Virol, 1997).

In a first step, ubqiuitin was amplified from a RNA preparation of murine B16 melanoma cells in a standard reverse-transcriptase-PCR (Titan One Tube RT-PCR System, Roche) according to the manufacturers instructions. The primers 5'-GGG CGG ATC CGA CCA TGC AGA TCT TCG TGA AGA CCC TGAC-3' and 5'-CAA AAC AGC CAG GAG CAT *CGC* ACC TCT CAG GCG AAG GAC CAG-3' were chosen in order to create a *Bam*HI restriction site (underlined) at the 5'-end of the resulting fragment and a 15 bp overlap to hTyr at the 3'-end and, furthermore, to mutate the ubiquitin residue G76 to A76 (residues latin and underlined).

In a second step, human tyrosinase was amplified by standard PCR from the plasmid pcDNAI-hTyr (Drexler *et al.* Cancer Res, 1999). The primers 5'-CGC CTG AGA GGT *GCG* ATG CTC CTG GCT GTT TTG TAC TGC CTG- 3' and 5'-GGG CGT TTA AAC TTA TAA ATG GCT CTG ATA CAA GCT GTG GT- 3' extended the hTyr cDNA with an 18 bp overlap to ubiquitin at the 5'-end and a PmeI restriction site at the 3'-end (underlined).

The resulting fragments were purified (PCR-purification-kit, Qiagen) and used as templates in a hybridization-PCR with the primers 5'-GGG CGG ATC CGA CCA TGC AGA TCT TCG TGA AGA CCC TGAC-3'and 5'-GGG CGT TTA AAC TTA TAA ATG GCT CTG ATA CAA GCT GTG GT- 3'. To create the MVA transfer vector pIIIdHR-P7.5-Ub-hTyr, the fused ubiquitin/tyrosinase gene (Ub-hTyr) was cloned into the unique BamHI/PmeI restriction site of pIIIdHR-P7.5, containing the P7.5 early/late promoter, *lacZ* gene sequences, the K1L host range selection gene and flanking MVA-DNA sequences for integration into the deletion III of the MVA genome.

The vector pIIIdHR-P7.5-Ub-hTyr was then transferred into Escherichia coli DH10B (Gibco) by electroporation and selected through resistance to ampicillin. Plasmid-DNA was amplified and prepared (Maxiprep Kit, Qiagen).

### Generation of Recombinant Viruses

Recombinant viruses were obtained by homologous recombination followed by transient host range selection as previously described (Staib et al. 2004). Briefly, monolayers of chicken embryo fibroblasts (CEF) were grown to 80% confluence in six-well tissue culture plates and then infected with MVA-wt at a multiplicity of infection (MOI) of 0.01 per cell. One hour post infection cells were transfected with the plasmid pIIIdHR-P7.5-Ub-hTyr using a transfection reagent (FuGENE6, Roche) and incubated for 8 hours in serum-free RPMI medium. Then cells were washed and harvested after 48h of incubation in RPMI/10%FCS, freeze-thawed 3x and sonicated in a cup sonicator. Liberated viruses were serially diluted and used to infect rabbit kidney cells (RK-13) for host-range-selection. Microscopically typical plaques were screened for the recombinant genes and wt-DNA after 2-3 plaque-passages on RK13 cells by DNA-extraction and PCR. MVA-wt free plaques producing recombinant MVA-Ub-hTyr were used to proceed with plaque purification on CEF cells to eliminate the K1L host range gene and then amplified. Viral stocks were purified, titered and analyzed by PCR for absence of MVA-wt and presence of MVA-Ub-hTyr.

### Western Blot analysis

Expression and degradation of ubiquitinated tyrosinase was confirmed by Western blot analysis. Baby hamster kidney cells (BHK), mouse fibroblasts (NIH 3T3), murine T lymphoma cells (RMA) and human cervix carcinoma cells (HeLa) were infected with an MOI=10 of recMVA encoding authentic (MVA-hTyr P7.5) or ubiquinated tyrosinase (MVA-Ub-hTyr P7.5) or parental MVA (MVA-wt) in the presence of specific proteasome inhibitors where indicated. Cells were harvested at indicated times, freeze-thawed and sonicated. Immunoprecipitation was performed where indicated (s.a. Fig. 8). Cell lysates were resolved by electrophoresis on a SDS-8% polyacrylamide gel and electroblotted onto nitrocellulose for 1 h in a buffer containing 25 mM Tris, 192 mM glycine, and 20% methanol (pH 8.6). The blots were blocked for 1 h at room temperature in a PBS blocking buffer containing 1% BSA and 0.1% NP40 and then incubated over night at room temperature with mAb T311 (Novocastra) diluted 100-fold in blocking buffer. After washing with 0.1% NP40 in PBS, the blots were incubated for 1 h at room temperature with horseradish-peroxidase-labeled anti-mouse IgG secondary antibody (Dianova), and visualized by enhanced chemoluminescence (ECL).

**Results:** Ubiquitinated tyrosinase expressed by MVA-Ub-Tyr is slightly bigger in size than authentic tyrosinase expressed by MVA-Tyr, reflecting the fusion to a 8kD-monoubiquitin. Ubiquitinated tyrosinase expressed by MVA-Ub-Tyr was only detectable in the presence of specific proteasome inhibitors, which did not affect expression or detection of authentic tyrosinase. There was no difference between the two constructs in the total amount of expressed protein when the proteasome was efficiently inhibited. Without proteasome inhibition, protein amount of ubiquitinated tyrosinase was below detection level of western blot analysis, indicating that ubiquitination of tyrosinase results in rapid proteasome-dependent degradation.

### Immunoprecipitation

Cell Lysates were prepared as described for Western Blot analysis and incubated with 0,2µg of mAb C-19 (Santa Cruz Biotech, Heidelberg) for 1 h at 4°C on a rocking device. Then 20µl of carefully shaked Protein-G-Agarose (Santa Cruz Biotech, Heidelberg) was added and probes were incubated over night at 4°C on a rocker.

### Pulse-Chase-Experiments and Radioimmunoprecipitation

Pulse-Chase-Experiments were performed with RMA cells infected with recMVA encoding authentic (MVA-hTyr P7.5) or ubiquinated tyrosinase (MVA-ubi/hTyr P7.5) or parental MVA (MVA-wt). 5 hours post infection cells were starved for 20 min with met/cys free Dubecco's medium containing ultraglutamin and pyruvat 1 % each, then pulsed for 45 min with 50µCi of ³⁵S-labeled methionine and cystein, and then chased with RPMI medium. Immunoprecipitation was performed at indicated times with anti-tyrosinase mAb C-19. Precipitates were separated by 8% SDS-PAGE and visualized on a phosphorimager.

**Results:** Pulse-Chase-Experiments showed similar amounts of radio-labeled proteins expressed by MVA-Tyr and MVA-Ub-Tyr. Again, ubiquitinated tyrosinase showed the expected increase in size. Authentic tyrosinase expressed by MVA-Tyr was stable over the 4h observation period, whereas ubiquitinated tyrosinase expressed by MVA-Ub-Tyr was subject to rapid degradation. The *in vivo* half life of ubiquitinated tyrosinase was significantly reduced and could be estimated to be less than 30 min. In contrast, and in concordance to our results, the in vivo half life of authentic tyrosinase has been estimated to be greater than 10 hrs (Jiménez et al., 1988)

### Chromium Release Assays

Specific lysis by A*0201-restricted murine CTL reactive against human tyrosinase peptide epitope 369-377 was determined in a 6-h [⁵¹Cr]-release assay. Briefly, HLA-A*0201-positive A375 cells were infected for 3 h with MVA-wt, MVA-hTyr or MVA-Ub-Tyr at an MOI of 5, washed once, labeled for 1 h at 37°C with 100 µCi Na⁵¹CrO₄, and then washed four times. Labeled target cells were plated in U-bottomed 96-well plates at 1 x 10⁴ cells/well and incubated for an additional 8 h at 37°C. Fifteen h after infection, effector cells were incubated with the target cells at various E:T ratios. After 6 h, 100 µl of supernatant per well were collected, and the specific ⁵¹Cr release was determined.

**Results:** Ubiquitination of tyrosinase resulted in significantly enhanced Tyr-specific CTL recognition of infected target cells indicating increased peptide processing and availability of higher MHC class I-peptide densities on the cell surface even at earlier timepoints during the viral infection.

### Mice and Vaccination Schedules

HLA-A*0201-transgenic HHD- or A2K^{b}-mice were derived from in-house breeding under specific pathogen-free conditions. Mice were vaccinated with indicated doses of rec MVA (i.p.) or DNA (i.m.). For acute phase tyrosinase-specific primary CD8+ T cell responses induced by single immunization mice were analyzed on day 8 post vaccination. For acute phase tyrosinase-specific secondary CD8+ T cell responses induced by MVA-MVA or DNA-MVA prime-boost immunizations mice were primed once (recMVA) and boosted on day 30 post prime or mice were primed twice (DNA) in a one week interval and boosted on day 30 after the first prime, and analyzed on day 5 after the last immunization. Mice were sacrified and the spleens were harvested to be analyzed by ICS or tetramer binding assays.

### Intracellular Cytokine Stain

Splenocytes from vaccinated mice were peptide stimulated with either the human tyrosinase epitope 369-377, the VV epitopes VP35#1 or B22R or the HER-2 epitope 435 as a control for 5 h; for the last 3 h, Brefeldin A (GolgiPlug, Pharmingen) was added. Intracellular cytokine staining for IFNγ production was performed by using the Cytofix/Cytoperm kit (Pharmingen) according to the manufacturer's recommendations. Data were acquired on a FACSCalibur or FACSCanto (both Becton Dickinson). Acquired data were further analyzed with FLOWJO (Tree Star) software.

### Phenotypical T Cell Analysis by MHC Tetramerstaining

Chimeric A2K^{b} tetramer reagents were generated as described (Busch et al. 1998). Cells were incubated with ethidium monazide (Molecular Probes) for live/dead discrimination and anti-Mouse-Fc-Ab to avoid unspecific binding of surface marker Abs, washed three times, followed by MHC tetramer and surface marker staining with mAbs anti-CD8α (clone 53-5.8) and anti-CD62L (clone MEL-14) (both Pharmingen) for 45 min and washed again three times. All steps were carried out at 4°C. Data were acquired on a FACSCalibur or FACSCanto (both Becton Dickinson). Acquired data were further analyzed with FLOWJO (Tree Star) software.

**Results:** Tyr-specific cytotoxic CD8+ T cell (CTL) responses elicited through a boost with MVA-Ub-hTyr showed an 2fold increase in Interferon-γ producing cells and an up to 3fold increase in tetramer binding cells in comparison to MVA-hTyr. This ability of MVA-Ub-hTyr to most efficiently enhance Tyr-specific CTL recall responses in mice had been primed with DNA- or MVA-hTyr was shown in both, A2K^{b}- and HHD-mice. Remarkably, MVA-Ub-hTyr could elicit strong recall responses after a prime vaccination of only 10e5 IU of MVA-hTyr in comparison to 10e7 IU which were necessary to elicit a comparable amount of Interferon-γ producing epitope specific CD8+ T cells when boosting with MVA-hTyr (Fig. 9).

In the present example the use of recMVA expressing a ubiquitinated antigen to elicit secondary immune responses allowed a up to 100fold reduction of viral doses for primary immunizations. This data show that the use of recMVA expressing ubiquitinated antigens to boost secondary immune responses can elicit stronger target antigen-specific cytotoxic CD8+ T cell responses at significantly lower viral doses. Importantly, this also demonstrated that the requirement of lower doses for priming was additonally reducing VV-specific CD8+ T cells in secondary responses against e.g. the VV epitope B22R.

### In vivo CTL assay

Autologous splenocytes of naive mice were prepared and divided into two groups. One group was pulsed with the human tyrosinase epitope 369-377 (1µM) and then labeled with a high concentration (5µM) of 5,6-carboxy-fluorescein succinimidyl ester (CFSE, Molecular Probes), the other group was pulsed with the HER-2 epitope 435-443 (1µM) as a control and then labeled with a low concentration of CFSE (0,5µM). For CFSE labeling 10⁷ peptide pulsed cells/ml PBS were incubated with CFSE at 37°C in a 5% CO2 incubator for 10 min. To stop the labeling reaction 20ml of RPMI/10%FCS was added. After washing three times with PBS to remove free CFSE, 1x10⁷ cells of each group were mixed 1:1 in 200µl of PBS per recipient mouse for intravenous injection via the tail vein. Spleens and blood from the recipient mice were harvested after 5 hours, and analyzed for specific lysis of target cells on a FACSCanto cytometer (Becton-Dickinson). At least 5,000 CFSE "low" labeled cells were aquired. The specific in vivo lysis was calculated as follows: 100 - ([(% CFSE "high" in vaccinated responder / CFSE "low" in vaccinated responder) / (% CFSE "high" in naive responder / CFSE "low" in naive responder)] x 100).

## Claims

1. A medicament including a kit of parts comprising the following components:
a) a first component comprising one or more foreign proteins or a nucleic acid encoding same or functional parts thereof, which functional parts contain one or more substitutions, insertions and/or deletions when compared to the wild type protein whilst having the same function as the wild type protein; and
b) a second component comprising a recombinant Modified Vaccinia Virus Ankara (MVA), which carries a nucleic acid sequence coding for a fusion protein comprising:
- Ubiquitin or a functional part thereof, which functional part contains one or more substitutions, insertions and/or deletions when compared to the wild type protein whilst having the same function as the wild type protein; and
- one or more foreign proteins, i.e. one or more proteins not naturally present as a part of MVA or functional parts thereof, which functional part contains one or more substitutions, insertions and/or deletions when compared to the wild type protein whilst having the same function as the wild type protein;
wherein the one or more foreign proteins are the same in a) and b), and
wherein the first and second components further comprise a pharmaceutically acceptable carrier.

2. The medicament of claim 1, wherein the fusion protein further comprises a linker between Ubiquitin and the foreign protein.

3. The medicament of claim 1 or 2, wherein the foreign protein is a heterologous protein derived from the group consisting of therapeutic polypeptides and polypeptides of pathogenic agents and functional parts thereof.

4. The medicament of claim 3, wherein the therapeutic polypeptide is derived from the group consisting of secreted proteins, e.g. polypeptides of antibodies, chemokines, cytokines or interferons, or wherein the pathogenic agent is derived from the group consisting of viruses, preferably selected from the group consisting of influenza viruses, measles and respiratory syncytial viruses, dengue viruses, human immunodeficiency viruses, human hepatitis viruses, herpes viruses, or papilloma viruses; bacteria, preferably tuberculosis-causing Mycobacteria; protozoa, preferably Plasmodium falciparum; and parasites as well as tumor cells or tumor cell associated antigens and functional parts thereof.

5. The medicament of claim 4, wherein the tumor cell associated antigen is selected from the group consisting of melanoma-associated differentiation antigens, e.g. tyrosinase, tyrosinase-related proteins 1 and 2, of cancer testes antigens, e.g. MAGE-1,-2,-3, and BAGE, and of non-mutated shared antigens overexpressed on tumors, e.g. Her-2/neu, MUC-1, and p53.

6. The medicament of one or more of claims 1-5, wherein the fusion protein and/or foreign protein coding regions are each flanked by DNA-sequences, flanking a non-essential site within the MVA genome, wherein the non-essential site preferably is the site of deletion III in the MVA genome.

7. The medicament of one or more of claims 1-6, wherein the ratio of the amount of the first component to the second component is from 1:5 to 1:20, preferably 1:10, measured as infectious units (IV) of recombinant MVA particles.

8. The medicament of one or more of claims 1-7 for use in the anti-cancer therapy or in the prevention of infectious diseases.

9. The medicament of one or more of the preceding claims, wherein the nucleic acids of component a) are carried by a viral vector.

10. The medicament of claim 9, wherein the vector is MVA.

11. A medicament as defined in one or more of claims 1-10 for use in a method for enhancing T cell responses in a mammal, preferably a human being, the method comprising the steps of:
a) Providing a medicament as defined in one or more of claims 1-10;
b) priming a mammal with an amount of the first component effective to provide a primary immune response;
c) boosting said mammal with an amount of the second component effective to provide a secondary immune response.

12. The medicament of claim 11 for use in the treatment of cancer or for use in the prevention of infectious diseases.

13. The medicament of claims 11 or 12, wherein the boosting step is performed at week 2 - 12, preferably 4-8 after the priming step.

14. The medicament of claims 11-13 for use in a vaccination method.

15. A recombinant MVA, which carries a nucleic acid sequence coding for a fusion protein comprising:
- Ubiquitin or a functional part thereof, which functional parts contain one or more substitutions, insertions and/or deletions when compared to the wild type protein whilst having the same function as the wild type protein; and
- one or more foreign proteins, i.e. one or more proteins not naturally present as a part of MVA or a functional part thereof, which functional part contains one or more substitutions, insertions and/or deletions when compared to the wild type protein whilst having the same function as the wild type protein,
for use in either anti-cancer therapy or in the prevention of infectious diseases as a boosting agent in prime-boost vaccinations.

## Patentansprüche

1. Medikament, das einen Kit of Parts einschließt, umfassend die folgenden Bestandteile:
a) einen ersten Bestandteil, umfassend ein oder mehrere Fremdproteine oder eine Nukleinsäure, die dieselben oder funktionelle Teile hiervon kodiert, wobei die funktionellen Teile eine oder mehrere Substitutionen, Insertionen und/oder Deletionen im Vergleich zum Wildtyp-Protein aufweisen, während sie dieselbe Funktion wie das Wildtyp-Protein aufweisen; und
b) einen zweiten Bestandteil, umfassend ein rekombinantes, modifiziertes Vaccinia Virus Ankara (MVA), das eine Nukleinsäuresequenz trägt, die für ein Fusionsprotein kodiert, das folgendes umfasst:
- Ubiquitin oder einen funktionellen Teil hiervon, wobei der funktionelle Teil ein oder mehrere Substitutionen, Insertionen und/oder Deletionen im Vergleich zum Wildtyp-Protein enthält, während er dieselbe Funktion wie das Wildtyp-Protein aufweist; und
- ein oder mehrere Fremdproteine, d.h. ein oder mehrere Proteine, die nicht natürlicherweise als Teil von MVA oder von funktionellen Teilen hiervon vorliegen, wobei die funktionellen Teile eine oder mehrere Substitutionen, Insertionen und/oder Deletionen im Vergleich zum Wildtyp-Protein aufweisen, während sie dieselbe Funktion wie das Wildtyp-Protein aufweisen;
wobei die ein oder mehreren Fremdproteine in a) und b) gleich sind, und
wobei die ersten und zweiten Bestandteile weiterhin einen pharmazeutisch verträglichen Träger umfassen.

2. Medikament nach Anspruch 1, wobei das Fusionsprotein weiterhin einen Linker zwischen Ubiquitin und dem Fremdprotein umfasst.

3. Medikament nach Anspruch 1 oder 2, wobei das Fremdprotein ein heterologes Protein ist, abgeleitet aus der Gruppe, die aus therapeutischen Polypeptiden und Polypeptiden von pathogenen Mitteln und funktionellen Teilen hiervon besteht.

4. Medikament nach Anspruch 3, wobei das therapeutische Polypeptid aus der Gruppe abgeleitet ist, die aus sezernierten Proteinen, beispielsweise Polypeptiden von Antikörpern, Chemokinen, Cytokinen oder Interferonen besteht, oder wobei das pathogene Mittel aus der Gruppe ausgewählt ist, die aus Viren, vorzugsweise aus der Gruppe von Influenzaviren, Masern und Respiratorischem-Synzytial-Viren, Dengue-Viren, humanen Immundefizienz-Viren, humanen Hepatitisviren, Herpesviren oder Papillomaviren; Bakterien, vorzugsweise Tuberkulose verursachenden Mycobakterien, Protozoen, vorzugsweise Plasmodium falciparum; und Parasiten ebenso wie Tumorzellen oder Tumorzell-assoziierten Antigenen und funktionellen Teilen hiervon besteht.

5. Medikament nach Anspruch 4, wobei das Tumorzell-assoziierte Antigen aus der Gruppe ausgewählt ist, die aus Melanom-assoziierten Differenzierungsantigenen, beispielsweise Tyrosinase, Tyrosinase-verwandten Proteinen 1 und 2, aus Tumor-Hoden-Antigenen, beispielsweise MAGE-1, -2, -3 und BAGE, und aus nicht-mutierten gemeinsamen Antigenen, überexprimiert auf Tumoren, besteht, beispielsweise Her-2/neu, MUC-1 und p53.

6. Medikament nach einem oder mehreren der Ansprüche 1 bis 5, wobei die Fusionsprotein und/oder Fremdprotein kodierenden Regionen jeweils durch DNA-Sequenzen flankiert sind, die einen nicht-essentiellen Ort innerhalb den MVA-Genoms flankieren, wobei der nicht-essentielle Ort vorzugsweise der Ort der Deletion III im MVA-Genom ist.

7. Medikament nach einem oder mehreren der Ansprüche 1 bis 6, wobei das Verhältnis der Menge des ersten Bestandteils zum zweiten Bestandteil von 1:5 bis 1:20, vorzugsweise 1:10, gemessen als infektiöse Einheiten (IU) rekombinanter MVA Partikel beträgt.

8. Medikament nach einem oder mehreren der Ansprüche 1 bis 7 zur Verwendung in der Anti-Krebstherapie oder in der Vorbeugung von Infektionskrankheiten.

9. Medikament nach einem oder mehreren der vorgehenden Ansprüche, wobei die Nukleinsäuren von Bestandteil a) von einem viralen Vektor getragen werden.

10. Medikament nach Anspruch 9, wobei der Vektor MVA ist.

11. Medikament wie in einem oder mehreren der Ansprüche 1 bis 10 definiert zur Verwendung in einem Verfahren zur Steigerung von T-Zellreaktionen in einem Säugetier, vorzugsweise einem Menschen, wobei das Verfahren die folgenden Schritte umfasst:
a) Bereitstellen eines Medikaments wie in einem oder mehreren der Ansprüche 1 bis 10 definiert;
b) Priming eines Säugetiers mit einer Menge des ersten Bestandteils der dazu wirksam ist, eine primäre Immunreaktion bereitzustellen;
c) Boosting des Säugetiers mit einer Menge des zweiten Bestandteils der dazu wirksam ist, eine sekundäre Immunreaktion bereitzustellen.

12. Medikament nach Anspruch 11 zur Verwendung in der Behandlung von Krebs oder zur Verwendung in der Vorbeugung von Infektionskrankheiten.

13. Medikament nach Ansprüchen 11 oder 12, wobei der Boosting-Schritt in Wochen 2 bis 12, vorzugsweise 4 bis 8 nach dem Priming-Schritt durchgeführt wird.

14. Medikament nach Anspruch 11 bis 13 zur Verwendung in einem Vakzinierungsverfahren.

15. Rekombinanter MVA, der eine Nukleinsäuresequenz trägt, kodierend für ein Fusionsprotein, das folgendes umfasst:
- Ubiquitin oder einen funktionellen Teil hiervon, wobei die funktionellen Teile ein oder mehrere Substitutionen, Insertionen und/oder Deletionen enthalten, wenn sie mit dem Wildtyp-Protein verglichen werden, während sie dieselbe Funktion wie das Wildtyp-Protein aufweisen; und
- ein oder mehrere Fremdproteine, d.h. ein oder mehrere Proteine, die nicht natürlicherweise als Teil von MVA oder eines funktionellen Teils hiervon vorliegen, wobei der funktionelle Teil ein oder mehrere Substitutionen, Insertionen und/oder Deletionen im Vergleich zum Wildtyp-Protein enthält, während dieselbe Funktion wie das Wildtyp-Protein aufrecht erhalten bleibt;
zur Verwendung entweder in der Anti-Krebstherapie oder in der Vorbeugung von Infektionskrankheiten als Boosting-Mittel in Prime-Boost-Vakzinierungen.

## Revendications

1. Médicament contenant plusieurs parties comprenant les composants suivants :
a) un premier composant qui comprend une ou plusieurs protéines étrangères ou un acide nucléique codant pour celle-ci ou pour des parties fonctionnelles de celle-ci, lesdites parties fonctionnelles comprenant une ou plusieurs substitutions, insertions et/ou délétions par rapport à la protéine de type sauvage tout en ayant la même fonction que la protéine de type sauvage, et
b) un deuxième composant qui comprend le virus recombinant de la vaccine modifiée souche Ankara (Modified Vaccinia Virus Ankara, MVA) qui porte une séquence d'acide nucléique codant pour une protéine de fusion comprenant :
- l'Ubiquitine ou une partie fonctionnelle de celle-ci, ladite partie fonctionnelle comprenant une ou plusieurs substitutions, insertions et/ou délétions par rapport à la protéine de type sauvage tout en ayant la même fonction que la protéine de type sauvage, et
- une ou plusieurs protéines étrangères, c'est-à-dire une ou plusieurs protéines qui ne sont pas naturellement présentes comme partie du MVA ou des parties fonctionnelles de celui-ci, ladite partie fonctionnelle comprenant une ou plusieurs substitutions, insertions et/ou délétions par rapport à la protéine de type sauvage tout en ayant la même fonction que la protéine de type sauvage,
dans lequel la ou les protéines étrangères sont identiques dans a) et dans b) et
dans lequel le premier et le deuxième composants comprennent en outre un véhicule pharmaceutiquement acceptable.

2. Médicament selon la revendication 1, dans lequel la protéine de fusion comprend en outre une séquence de liaison entre l'Ubiquitine et la protéine étrangère.

3. Médicament selon la revendication 1 ou 2, dans lequel la protéine étrangère est une protéine hétérologue dérivée du groupe constitué par les polypeptides thérapeutiques et les polypeptides d'agents pathogènes et les parties fonctionnelles de ceux-ci.

4. Médicament selon la revendication 3, dans lequel le polypeptide thérapeutique est dérivé du groupe constitué par les protéines sécrétées, par exemple les polypeptides d'anticorps, les chimiokines, les cytokines ou les interférons ou dans lequel l'agent pathogène est dérivé du groupe constitué par les virus, de préférence choisis parmi le groupe constitué par les virus de la grippe, les virus de la rougeole et les virus respiratoires syncytiaux, les virus de la dengue, les virus de l'immunodéficience humaine, les virus de l'hépatite humaine, les virus de l'herpès ou les papillomavirus ; les bactéries, de préférence les mycobactéries responsables de la tuberculose ; les protozoaires, de préférence Plasmodium falciparum ; et les parasites de même que des cellules tumorales ou des antigènes associés à la cellule tumorale et des parties fonctionnelles de ceux-ci.

5. Médicament selon la revendication 4, dans lequel l'antigène associé à la cellule tumorale est choisi parmi le groupe constitué par les antigènes de différenciation associés au mélanome, par exemple la tyrosinase, les protéines 1 et 2 liées à la tyrosinase, par des antigènes contre le cancer des testicules, par exemple MAGE-1,-2,-3, et BAGE, et par des antigènes partagés non mutés surexprimés sur les tumeurs, par exemple Her-2/neu, MUC-1 et p53.

6. Médicament selon une ou plusieurs des revendications 1 à 5, dans lequel chacune des régions codant pour la protéine de fusion et/ou la protéine étrangère est flanquée de séquences d'ADN qui flanquent un site du génome de MVA non essentiel, le site non essentiel étant de préférence un site de délétion III dans le génome de MVA.

7. Médicament selon une ou plusieurs des revendications 1 à 6, dans lequel le rapport entre la quantité du premier composant et celle du deuxième composant est compris entre 1 : 5 et 1 : 20, de préférence 1:10, mesuré comme unités infectieuses (IU) des particules de MVA recombinantes.

8. Médicament selon une ou plusieurs des revendications 1 à 7, à utiliser dans la thérapie anti-cancéreuse ou dans la prévention de maladies infectieuses.

9. Médicament selon une ou plusieurs des revendications précédentes, dans lequel les acides nucléiques du composant a) sont portés par un vecteur viral.

10. Médicament selon la revendication 9, dans lequel le vecteur est le MVA.

11. Médicament tel qu'il est défini dans une ou plusieurs des revendications 1 à 10, à utiliser dans une méthode destinée à favoriser les réponses des lymphocytes T chez un mammifère, de préférence un être humain, ladite méthode comprenant les étapes suivantes :
a) formuler un médicament tel qu'il est défini dans une ou plusieurs des revendications 1 à 10,
b) faire une première immunisation du mammifère à l'aide d'une quantité efficace du premier composant pour provoquer une réaction immunitaire primaire,
c) faire un rappel audit mammifère à l'aide d'une quantité efficace du deuxième composant pour provoquer une réaction immunitaire secondaire.

12. Médicament selon la revendication 11, à utiliser dans la thérapie anti-cancéreuse ou dans la prévention de maladies infectieuses.

13. Médicament selon les revendications 11 ou 12, dans lequel l'étape de rappel est réalisée de 2 à 12 semaines, de préférence de 4 à 8 semaines, après l'étape de première immunisation.

14. Médicament selon les revendications 11 à 13, à utiliser dans une méthode de vaccination.

15. MVA recombinant, lequel porte une séquence d'acide nucléique codant pour une protéine de fusion comprenant :
- l'Ubiquitine ou une partie fonctionnelle de celle-ci, ladite partie fonctionnelle comprenant une ou plusieurs substitutions, insertions et/ou délétions par rapport à la protéine de type sauvage tout en ayant la même fonction que la protéine de type sauvage, et
- une ou plusieurs protéines étrangères, c'est-à-dire une ou plusieurs protéines qui ne sont pas naturellement présentes comme partie du MVA ou des parties fonctionnelles de celui-ci, ladite partie fonctionnelle comprenant une ou plusieurs substitutions, insertions et/ou délétions par rapport à la protéine de type sauvage tout en ayant la même fonction que la protéine de type sauvage,
pour utilisation soit dans la thérapie anti-cancéreuse soit dans la prévention de maladies infectieuses comme agent de rappel dans des primo-vaccinations-rappel.
